# FASCICULE DE BREVET EUROPEEN

(11) **EP 3 281 666 B1**
(45) Date de publication et mention de la délivrance du brevet: **01.01.2020**
(21) Numéro de dépôt: 16197160.1
(22) Date de dépôt: 03.11.2016
(51) Int. Cl.: A61M 21/02, A63F 13/26, A63F 13/54, A61M 21/00

(54) **SYSTÈME DE RÉALITÉ VIRTUELLE**
VIRTUELLES REALITÄTSSYSTEM
VIRTUAL REALITY SYSTEM

(30) Priorité: 10.08.2016 CH 10312016
(43) Date de publication de la demande: 14.02.2018
(73) Titulaire: E-PNOgraphic Sàrl, 1134 Chigny (CH)
(72) Inventeur: DERUNGS, Louis, 1134 Vufflens-le-Chãteau (CH)
(74) Mandataire: P&TS SA (AG, Ltd.)

(56) Documents cités:
- WO-A2-2013/049248
- US-A1- 2006 252 979
- US-A1- 2013 170 650
- US-A1- 2014 316 191

## Description

### Domaine technique

La présente invention concerne un système de réalité virtuelle.

### Etat de la technique

Il est connu l'utilisation de dispositif supportant un utilisateur ou un patient de sorte à le conduire dans un état de conscience altérée profonde jusqu'à un état d'hypnose profonde.

Le document US8517912 décrit un dispositif d'hypnose médicale pour contrôler une expérience d'hypnose chez un patient. Le dispositif comprend des moyens de sortie pour fournir un premier type de contenu qui peut être une représentation de type réalité virtuelle.

Le document US2006247489 décrit un dispositif et une méthode de relaxation et méditation par hypnose dans un environnement virtuel. Le dispositif comprend un casque audiovisuel connectable à un lecteur DVD portable pour reproduire des films à partir de supports DVD de sorte à induire l'utilisateur dans un état de relaxation jusqu'à un état d'hypnose profond. Le dispositif peut comprendre un moniteur 2D ou 3D.

Le document US2015174362 décrit un appareil comprenant un module de réalité virtuelle et une méthode d'induction d'un état d'hypnose. L'appareil comprend un casque de visualisation permettant de délivrer un signal audiovisuel de réalité virtuelle au patient ainsi qu'une collecte de ses signaux biologique.

Le document US2006252979 décrit un système de lunettes a rétroaction biologique comprenant des lentilles stéréo, un système audio binaural et plusieurs électrodes destinées aux dispositifs à rétroaction biologique. Le système audio binaural comprend des écouteurs gauche et droit, ce qui permet à l'utilisateur d'entendre un son en 3D.

Le document US2014316191 décrit un système de réalité virtuelle à biofeedback pour surveiller un ou plusieurs paramètres physiologiques tout en présentant un environnement immersif. La présentation de l'environnement immersif change au fil du temps en réponse aux changements dans les valeurs des paramètres physiologiques.

Le document US2013170650 décrit un générateur conçu pour générer plusieurs éléments de données sonores originales indiquant chacune un son original en tant que base pour qu'un son traité soit entendu par un utilisateur et un ensemble individuel de paramètres indiquant le contenu de traitement à effectuer sur chaque élément des données sonores originales.

### Bref résumé de l'invention

La présente invention concerne un système de réalité virtuelle, comprenant dispositif de réalité virtuelle comportant un module de réalité virtuelle comportant un dispositif de visualisation configuré pour projeter d'une séquence d'image dans le champ visuel d'un utilisateur, un dispositif de reproduction audio configuré pour produire un premier signal sonore comprenant une bande sonore, un second signal sonore ayant une première fréquence et audible d'une oreille de l'utilisateur, un troisième signal sonore ayant une seconde fréquence et audible de l'autre oreille de l'utilisateur, et un quatrième signal sonore comprenant un exposé oratoire, et une unité de contrôle configurée pour contrôler le module de réalité virtuelle;
le dispositif de visualisation étant configuré pour projeter la séquence d'images, et le dispositif de reproduction audio étant configuré pour produire le premier signal sonore, le second signal sonore, le troisième signal sonore et le quatrième signal sonore pendant une période de stimulation comportant une portion initiale, portion médiane et une portion finale; et
le dispositif de visualisation étant configuré pour produire en outre un signal d'induction pendant la portion initiale, le signal d'induction comprenant une projection d'un signal lumineux superposé à la séquence d'images, ou un son bi-canal se déplaçant rythmiquement et selon un mouvement de va-et-vient entre une première et une deuxième zone de l'espace entourant l'utilisateur.

La présente invention concerne également un support informatique comprenant des portions de code d'un programme d'application destinées à être exécutées par le système de réalité virtuelle de manière à projeter la séquence d'images et de produire le premier signal sonore, le second signal sonore, le troisième signal sonore et le quatrième signal sonore pendant une période de stimulation comportant une portion initiale, portion médiane et une portion finale; et de produire un signal d'induction pendant la portion initiale, le signal d'induction comprenant une projection d'un signal lumineux superposé à la séquence d'images ou un son bi-canal se déplaçant rythmiquement et selon un mouvement de va-et-vient entre une première et une deuxième zone de l'espace entourant l'utilisateur.

Ces solutions présentent notamment l'avantage par rapport à l'art antérieur de fournir des prestations de relaxation et méditation plus performantes par rapport à l'état de l'art.

### Brève description des figures

Des exemples de mise en œuvre de l'invention sont indiqués dans la description illustrée par les figures annexées dans lesquelles:
la figure 1 illustre schématiquement un système de réalité virtuelle comprenant un dispositif de réalité virtuelle et un dispositif de visualisation, selon un mode de réalisation;
la figure 2 représente une méthode destinée à être mise en œuvre dans le système de réalité virtuelle, selon un mode de réalisation;
la figure 3 montre le système de réalité virtuelle selon une forme de réalisation particulière;
la figure 4 montre le dispositif de visualisation, selon un mode de réalisation;
la figure 5 illustre un exemple du dispositif de visualisation, selon un autre mode de réalisation; et
la figure 6 montre une variante de réalisation du système de réalité virtuelle.

### Exemple(s) de mode de réalisation

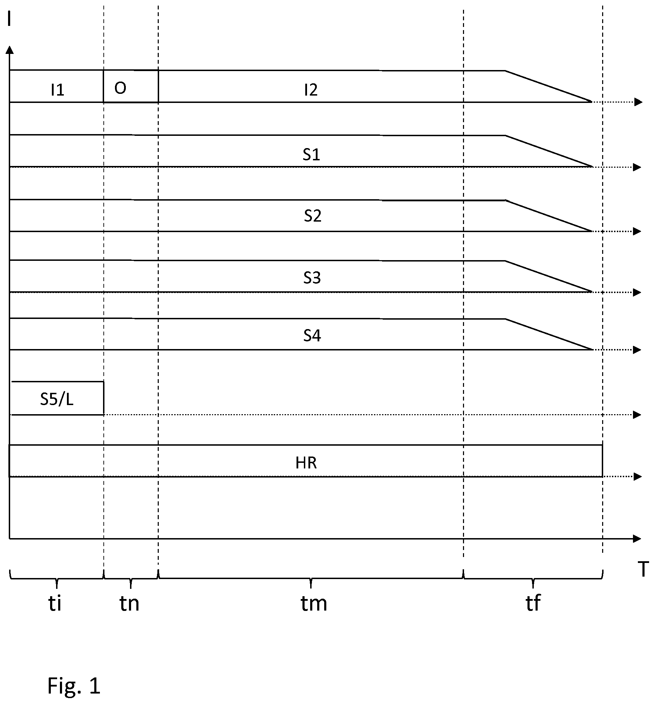

La figure 1 représente une système de réalité virtuelle selon un mode de réalisation. La méthode comprend la production d'un stimuli pendant une période de stimulation. Le stimuli peut comprendre une projection d'une séquence d'images I1. Le stimuli peut également comprendre la production du premier signal sonore S1 comprenant une bande sonore liée au déroulement de la séquence d'images I1. Le stimuli peut également comprendre la production du second signal sonore S2 ayant la première fréquence f1 et du troisième signal sonore S3 ayant la seconde fréquence f2. Le second signal sonore est audible d'une oreille d'un l'utilisateur et le troisième signal sonore étant audible de l'autre oreille de l'utilisateur. Le stimuli peut également comprendre la production d'un quatrième signal sonore S4 comprenant un exposé oratoire.

Pendant une portion initiale ti de la période de stimulation, le stimuli comprend en outre un signal d'induction S5. Pendant une portion finale tf de la période de stimulation, l'intensité des signaux sonores S1-S4 diminuent d'intensité jusqu'à une intensité nulle, et la séquence d'image I1 diminuant d'intensité jusqu'à une intensité nulle.

Selon une forme d'exécution, la première fréquence f1 du second signal sonore S2 diffère de la seconde fréquence f2 du troisième signal sonore S3. Par exemple, la différence entre la première et la seconde fréquence f1, f2 peut être constante sur une portion ou la totalité de la période de stimulation. En particulier, la différence entre la première et la seconde fréquence f1, f2 est comprise entre 5 Hz et 12 Hz.

Selon une forme d'exécution, la première et la seconde fréquence f1, f2 varient pendant la portion ou la totalité de la période de stimulation, de manière à ce que la différence entre la première et la seconde fréquence f1, f2 reste constante. Typiquement, la première et la seconde fréquences f1, f2 sont comprises entre 20 Hz et 600 Hz.

Dans un mode de réalisation, le quatrième signal sonore S4 comprend un exposé oratoire formant un discourt à caractère hypnotique. Le quatrième signal sonore S4 peut être préenregistré ou récité par un opérateur au travers d'un dispositif en communication avec le système de réalité virtuel 1. L'exposé oratoire permet d'influencer et de conduire l'utilisateur 10 vers un état de relaxation et de médiation.

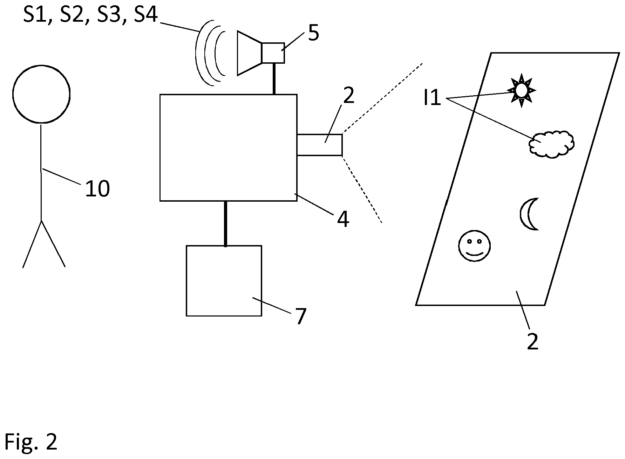

La figure 2 illustre schématiquement un système de réalité virtuelle 1 selon un mode de réalisation. Le système 1 comprend un module de réalité virtuelle 4 comportant un dispositif de visualisation 2 configuré pour projeter d'une séquence d'image I1 dans le champ visuel d'un utilisateur 10. Le module de réalité virtuelle 4 comprend également un dispositif de reproduction audio 5 configuré pour produire le premier signal sonore S1, le second signal sonore S2 ayant la première fréquence f1 et audible d'une oreille de l'utilisateur, le troisième signal sonore S3 ayant la seconde fréquence f2 et audible de l'autre oreille de l'utilisateur, et le quatrième signal sonore S4 comprenant un exposé oratoire. Le système 1 comprend également une unité de contrôle 7 configurée pour contrôler le module de réalité virtuelle 4. La projection de la séquence d'images I1 avec la bande sonore S1 au moyen du dispositif de visualisation 2 et le dispositif de reproduction audio 5 du système de réalité virtuelle 1 permet de simuler la présence de l'utilisateur 10 dans l'environnement réel ou imaginaire projeté par la séquence d'images I1 (par exemple un film). Le système de réalité virtuelle 1 peut permettre une reproduction de films sonorisés ou de séquences d'images sonorisées et personnalisées, notamment sur demande de l'utilisateur 10.

Le système de réalité virtuelle 1 peut être adapté à ce que le second signal sonore S2 et le troisième signal sonore S3 soient respectivement audible uniquement par l'une ou l'autre oreille de l'utilisateur 10.

La projection de la séquence d'images I1 mise en œuvre par le système de réalité virtuelle 1 permet à l'utilisateur d'interagir spatialement avec l'environnement projeté, par exemple en modifiant l'angle de vue de l'environnement en modifiant sa posture, par exemple en tournant sa tête ou son buste.

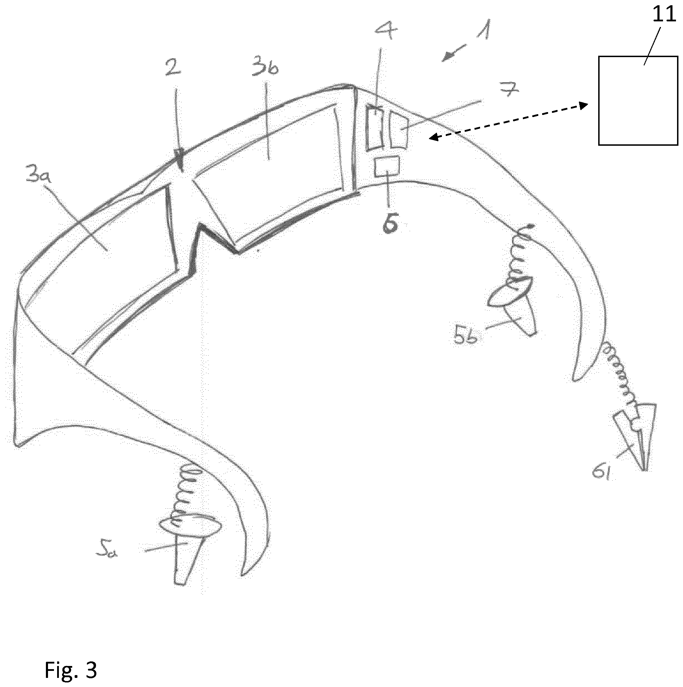

La figure 3 montre le système de réalité virtuelle 1 selon une forme de réalisation particulière dans laquelle le système 1 prend la forme de lunettes 1 portée par l'utilisateur. Le dispositif de visualisation 2 comprend deux écrans d'affichage 3a, 3b correspondants au verres des lunettes de sorte que chaque écran d'affichage 3a, 3b est dans le champ visuel d'un des deux yeux de l'utilisateur 10, lorsque le système 1 est porté par ce dernier.

Selon une forme d'exécution, le dispositif de reproduction audio 5 comprend deux transducteurs 5a, 5b, par exemple sous la forme d'oreillettes. Chaque transducteur 5a, 5b permet la production du second signal sonore S2 et du troisième signal sonore S3 dans chacune des oreilles de l'utilisateur 10.

Dans un mode de réalisation, pendant une portion médiane tm subséquente à la portion initiale ti et précédent la portion finale tf, la production de la séquence d'images I1 permet une interaction de l'utilisateur avec la séquence d'images I1. Par exemple, des caractéristiques de la séquence d'images I1, notamment l'intensité lumineuse, sont modifiées par l'interaction de l'utilisateur 10.

La portion médiane tm peut donc permettre à l'utilisateur d'interagir avec la séquence d'images I1 et/ou avec le quatrième signal sonore S4.

Par exemple, la séquence d'images I1 peut comprendre un ou une pluralité d'objets virtuels qui apparaissent et/ou disparaissent suite à l'interaction de l'utilisateur 10. L'interaction de l'utilisateur 10 peut comprendre comprend un mouvement de l'utilisateur en relation avec l'objet. Alternativement, le mouvement de l'utilisateur 10 peut comprendre une direction du regard de ses yeux et/ou une orientation de sa tête (par exemple dans la direction de ou des objets) pendant un période de temps prédéterminée.

Le dispositif de visualisation 2 peut être arrangé de manière à ce que la séquence d'image I1 produite permette une interaction de l'utilisateur 10. Dans ce but, le dispositif de visualisation 2 peut être configuré pour modifier les caractéristiques de la séquence d'images I1, par exemple l'intensité lumineuse des images, suite à une interaction de l'utilisateur 10.

Avantageusement, le dispositif de visualisation 2 est configuré de manière à faire apparaitre et/ou disparaitre un ou une pluralité d'objets virtuels compris dans la séquence d'images I1, suite à une interaction de l'utilisateur 10. L'apparition et/ou la disparition du ou des objets virtuels peut se produire dans une période de temps prédéfinie après l'interaction, de sorte à induire un état d'âme bénéfique dans l'utilisateur.

L'utilisateur 10 peut être conduit, par moyen de l'exposé oratoire S4, à indiquer un ou des objets virtuels avec lequel il interagit. L'exposé oratoire S4 peut également servir à repérer le ou les objets virtuels avec lesquels l'utilisateur 10 va interagir. L'interaction peut également être réalisée oralement par l'utilisateur 10 seulement ou en plus du mouvement de l'utilisateur 10.

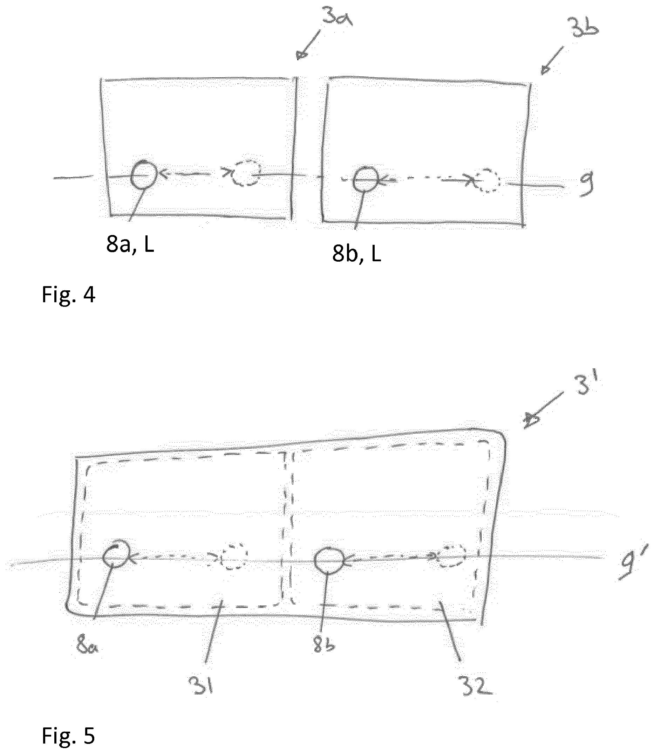

Selon une forme d'exécution, pendant la portion initiale ti de la période de stimulation, le signal d'induction S5 comprend une projection d'un signal lumineux L superposé à la séquence d'images I1 (voir la figure 4). Le signal lumineux L peut se déplacer selon un mouvement aléatoire ou prédéterminé entre une première et une deuxième zone de la séquence d'images I1. Dans un variante, le signal lumineux L se déplace rythmiquement selon un mouvement de va-et-vient entre une première et une deuxième zone de la séquence d'images I1. Le signal d'induction S5 a pour but de faciliter le passage de l'utilisateur à un état de relaxation et de méditation, voire d'hypnose.

Pendant la période initiale ti, la projection de la séquence d'images I1 avec la bande sonore S1 peut prendre un caractère de décor, ce qui favorise la relaxation chez l'utilisateur 10. La séquence d'images I1 peut ainsi permettre à l'utilisateur 10 de visualiser une succession de fonds. De plus, un ou plusieurs motifs lumineux (tel un point ou autre) peuvent se superposer sur la séquence d'images. chaque motif lumineux peut avoir une couleur distincte et uniforme. Ces images, possiblement avec le ou les motifs lumineux, permettent, par exemple, une mise en œuvre d'un décor clame et relaxant et/ou des techniques de chromothérapie.

A l'issue de la période initiale ti, la production du signal d'induction S5 cesse.

La figure 4 illustre le dispositif de visualisation 2 configuré pour visualiser simultanément un premier objet virtuel 8a (ou un signal lumineux L) par l'intermédiaire du premier écran d'affichage 3a et un deuxième objet virtuel 8b (ou un autre signal lumineux L) par l'intermédiaire de deuxième écran d'affichage 3b. Un tel arrangement permet de générer un effet de vision stéréoscopique, c'est-à-dire, de reproduire une perception d'un relief de l'objet virtuel 8a, 8b à partir de deux images planes illustrés par les deux écrans 3a, 3b. Ces objets virtuels peuvent être superposés sur la séquence d'images I1.

Le premier objet virtuel 8a peut se déplacer rythmiquement et selon un mouvement de va-et-vient entre une première et une deuxième zone du premier écran 3a. De manière similaire, le deuxième objet virtuel 8b peut se déplacer rythmiquement et selon un mouvement de va-et-vient entre une première et une deuxième zone du deuxième écran 3b.

Dans l'exemple de la figure 4, le premier objet virtuel 8a et le deuxième objet virtuel 8b se déplacent le long d'une ligne de déplacement 9 reliant virtuellement le premier écran 3a et le deuxième écran 3b de sorte à déplacer les objets 8a, 8b entre une portion périphérique droite et une portion périphérique gauche du champ de vision de chaque œil. Une telle configuration permet, par exemple, une mise en œuvre de protocoles d'implémentation de type EMDR développé par Shapiro.

Avantageusement, le dispositif de visualisation 2 peut être configuré pour varier dynamiquement l'angle de la ligne de déplacement 9 (par rapport à la disposition géométrique des écrans 3a, 3b) en réponse à l'inclinaison latérale du système 1, lorsqu'il est porté par l'utilisateur 10. La ligne de déplacement 9 peut ainsi être maintenue sensiblement parallèle à l'horizontale (c'est-à-dire perpendiculaire à la direction de la gravité).

Selon une autre forme d'exécution, le signal d'induction comprend une production d'un cinquième signal sonore S5. Le cinquième signal sonore S5 peut se déplacer selon un mouvement aléatoire ou prédéterminé. Dans un variante, le cinquième signal sonore S5 se déplace selon un mouvement de va-et-vient entre une première et une deuxième zone dans l'espace entourant l'utilisateur.

Selon une forme d'exécution, le dispositif de reproduction audio 5 peut être configuré de manière à ce que le signal d'induction S5 comprend un son bi-canal se déplaçant rythmiquement et selon un mouvement de va-et-vient entre une première et une deuxième zone de l'espace entourant l'utilisateur 10. De manière préférée, la source sonore virtuelle S5 peut se déplacer le long d'une ligne reliant virtuellement le premier et le deuxième transducteur 5a, 5b (voir la figure 3). Cette dernière configuration permet une mise en œuvre de protocoles d'implémentation de type EMDR développé par Shapiro.

En particulier, le système de réalité virtuelle 1 est adapté a mise en œuvre d'une méthode de réalité virtuelle qui requiert de l'utilisateur 10 de réaliser une séquence de mouvements oculaires, ceci en combinaison avec des signaux sonores et/ou des séquences d'images prédéterminées qui peuvent produire des décors ou autres environnements visuels.

Dans un mode de réalisation, la période de stimulation comprend une portion noire tn subséquente à la portion initiale ti et précédent la portion médiane tm, dans laquelle la séquence d'images consiste en une obscurité totale O. Pendant la période noire tn, la séquence d'images O simule un environnement d'obscurité totale, par exemple par une suite d'images noire ou par une diminution de l'intensité des images jusqu'à une intensité nulle ou perçu comme nulle. La période noire tn peut être utilisée pour renforcer et vérifier l'état de relaxation et de méditation chez l'utilisateur 10.

Encore dans un autre mode de réalisation, le système de réalité virtuelle 1 comprend au moins un module capteur 6 configuré pour mesurer un paramètre vital HR (biofeedback) de l'utilisateur 10. Par exemple, le module capteur 6 peut comprendre un capteur permettant de fournir un signal cardiovasculaire, permettant par exemple de déterminer la cohérence cardiaque de l'utilisateur 10. Un tel capteur peut comprendre un dispositif optique tel qu'un capteur photopléthysmographique ou encore un capteur de type ECG ou ICG (cardiographie par impédance). La figure 3 illustre un module capteur photopléthysmographique prenant la forme d'un clip 61 (d'oreille).

L'unité de contrôle 7 peut être configurée pour contrôler le capteur 6 et possiblement collecter et analyser la signal cardiovasculaire fournie par le capteur 6, par exemple de manière à déterminer une cohérence cardiaque ainsi qu'un seuil de cohérence cardiaque.

Selon une forme d'exécution, le système détermine la cohérence cardiaque de l'utilisateur 10 à l'aide du signal cardiovasculaire fourni par le module capteur 6, pendant la portion médiane tm. Le système peut revenir à la portion initiale ti de la période de stimulation dans laquelle le signal d'induction S5 est généré afin de remettre l'utilisateur dans l'état souhaité (par exemple en cas d'état de relaxation insuffisante ou absente), lorsque la cohérence cardiaque déterminée est sous le seuil de cohérence cardiaque. La période initiale ti peut être prolongé ou répétée de manière à induire un état de relaxation et de méditation suffisant de l'utilisateur.

L'unité de contrôle 7 peut comprendre un module de transmission (non représenté) agencé pour permettre une transmission des données collectées par l'unité de contrôle 7 vers un module externe 11. Le module externe 11 peut être configuré pour réaliser le traitement et/ou la visualisation et/ou de stockage des données collectées. Le module externe 11 peut être également arrangé pour recevoir des commandes et/ou des images ou séquences d'images destinées à être générées par le dispositif de visualisation 2 et/ou à recevoir des commandes et/ou des sons destinés à être générés le dispositif de reproduction audio 5.

Avantageusement, l'unité de contrôle 7 peut être configurée pour recevoir des messages vocaux de la part d'un tiers de sorte à les reproduire au travers du dispositif de reproduction audio 5.

En particulier, l'unité de contrôle 7 peut être configurée pour générer une base de données de séquences d'images et accéder à la base de données de sorte à permettre à l'utilisateur de télécharger du nouveau contenu visuel afin d'être reproduit avec le système 1.

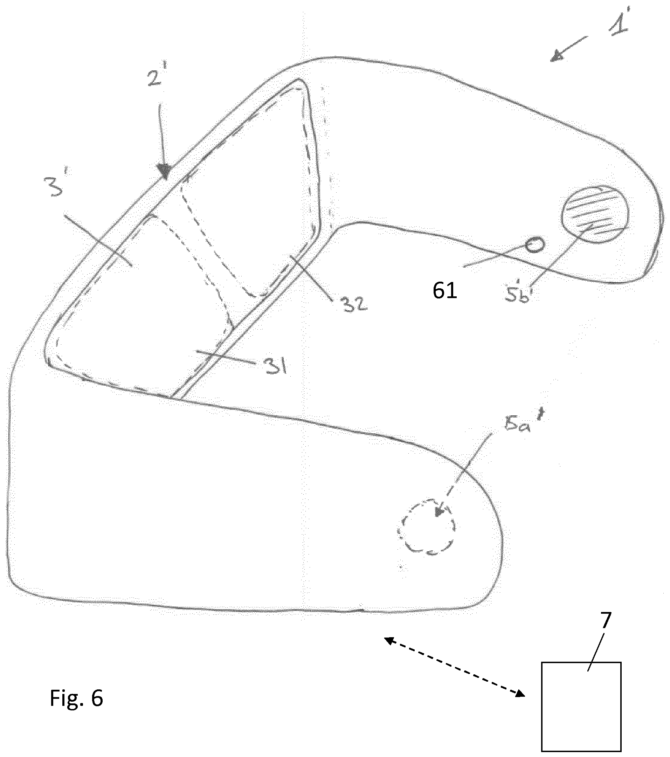

La figure 6 illustre une variante de réalisation du système de réalité virtuelle 1. Ici, le système 1 portable prend la forme d'un visiocasque (parfois aussi appelé casque de visualisation, casque immersif, casque de réalité virtuelle, casque-écran ou casque HMD). Cette variante du système 1 reprend les caractéristiques techniques du système 1 de la figure 3. Cependant, le dispositif de visualisation 2 comprend un seul écran d'affichage 3 couvrant les champs de vision des deux yeux de l'utilisateur. Une première portion 31 de l'écran d'affichage 3 est positionnée dans le champ visuel du premier œil de l'utilisateur, et une deuxième portion 32 de l'écran est positionnée dans le champ visuel du deuxième œil de l'utilisateur, lorsque ce dernier porte le système 1.

Le module de réalité virtuelle 4 peut être configuré pour visualiser le premier objet virtuel 8a et/ou le deuxième objet virtuel 8b par l'intermédiaire du même écran d'affichage 3. Avantageusement, le module de réalité virtuelle 4 est configuré pour visualiser le premier objet virtuel 8a se déplaçant dans la première portion 31 de l'écran 3 et simultanément le deuxième objet 8b se déplaçant dans la deuxième portion 32 de l'écran 3 de sorte à générer un effet de vision stéréoscopique.

En particulier, le module de réalité virtuel 4 peut être configuré pour visualiser le premier objet virtuel 8a se déplaçant rythmiquement et selon un mouvement de va-et-vient entre une première et une deuxième zone de la première portion 31 d'écran 3. Le module de réalité virtuel 4 peut également être configuré pour visualiser le deuxième objet virtuel 8b se déplaçant rythmiquement et selon un mouvement de va-et-vient entre une première et une deuxième zone de la deuxième portion 32 d'écran 3.

Le dispositif de visualisation 2 est arrangé de sorte que chaque portion de l'écran 3a, 3b, 31, 32 soit positionnée uniquement dans un des deux champs de vision des yeux de l'utilisateur.

La figure 5 illustre un exemple dans lequel le dispositif de visualisation 2 est configuré pour que le premier objet virtuel 8a et le deuxième objet virtuel 8b se déplacent le long d'une ligne de déplacement 9 reliant virtuellement les deux bords latéraux de l'écran d'affichage 3. Le module de réalité virtuel module permet ainsi une mise en œuvre de protocoles d'implémentation de type EMDR développé par Shapiro.

Avantageusement, le module de réalité virtuel peut être configuré pour varier dynamiquement l'angle de la ligne de déplacement 9 (par rapport aux bords latéraux de l'écran d'affichage 3) en réponse à l'inclinaison latérale du système 1, lorsqu'il est porté par l'utilisateur 10. La ligne de déplacement 9 peut ainsi être maintenue sensiblement parallèle à l'horizontale (c'est-à-dire perpendiculaire à la direction de la gravité).

Le système 1 selon la variante de la figure 6 permet une intégration du premier et deuxième transducteur 5a, 5b du dispositif de reproduction audio stéréophonique. Les transducteurs 5a, 5b peuvent ainsi prendre la forme de hautparleurs. Le système 1 permet une intégration facile du module capteur 6.

Selon une forme d'exécution illustrée aux figures 1 et 6, l'unité de contrôle 7 est distante du dispositif de visualisation 2. La totalité ou une partie du module de réalité virtuelle 4 peut également être distant du dispositif de visualisation 2. L'unité de contrôle 7 (possiblement aussi la totalité ou une partie du module de réalité virtuelle 4) et le module capteur 6 communiquent entre eux à l'aide d'un protocole de communication sans fil. Dans une variante, l'unité de contrôle 7 et possiblement aussi la totalité ou une partie du module de réalité virtuelle 4 sont compris dans un appareil portable tel qu'un smartphone, une tablette, etc.

Le système peut collecter initialement des informations par l'intermédiaire du système de réalité virtuel 1. Ces informations peuvent être collectées au travers d'un questionnaire virtuel de sorte à permettre une calibration des divers étapes de la méthode (intensité des signaux sonores S1-S5 et des images I1) ainsi que la fixation de ses périodes (ti, tn, tm, tf). Le système de réalité virtuel 1 peut comprendre des moyens vocaux, tels qu'un microphone, ou optiques (caméra) ou tous autres moyens appropriés à la collecte d'information.

Le système peut être adaptée à la préparation sportive, l'augmentation du rendement physique d'un sportif, le développement personnel, la relaxation, la médiation, la désensibilisation au tabagisme ou à des habitudes néfastes alimentaires, ainsi que la préparation aux opérations dentaires et orthopédiques de courte durée, de remplacement des anesthésies générales de confort et de pose d'un anneau gastrique virtuel.

En particulier, le système peut être adaptée à l'hypnose, par exemple une combinaison d'hypnose et d'une technique de type EMDR (Eye movement desensitization and reprocessing) et/ou EFT (Emotional Freedom Technique).

La présente invention concerne également un support informatique comprenant des portions de code d'un programme d'application destinées à être exécutées par l'unité de contrôle 7.

### Numéros de référence employés sur les figures

- 1: système de réalité virtuelle
- 2: dispositif de visualisation
- 3: écran d'affichage
- 3a: premier écran
- 3b: deuxième écran
- 31: première portion de l'écran d'affichage
- 32: deuxième portion de l'écran d'affichage
- 4: module de réalité virtuelle
- 5: dispositif de reproduction audio
- 5a,: premier transducteur
- 5b: deuxième transducteur
- 6: module capteur
- 61: clip
- 7: unité de contrôle
- 8a: premier objet virtuel
- 8b: deuxième objet virtuel
- 9: ligne de de déplacement
- 10: utilisateur
- 11: module externe
- I1: séquence d'images
- f1: première fréquence
- f2: seconde fréquence
- HR: paramètre vital
- L: signal lumineux
- O: obscurité totale
- S1: premier signal sonore
- S2: second signal sonore
- S3: troisième signal sonore
- S4: quatrième signal sonore
- S5: signal d'induction
- tf: portion finale
- ti: portion initiale
- tm: portion médiane
- tn: portion noire

## Revendications

1. Système de réalité virtuelle, comprenant:
un dispositif de réalité virtuelle (1) comportant un module de réalité virtuelle (4) comportant un dispositif de visualisation (2) configuré pour projeter d'une séquence d'image (I1) dans le champ visuel d'un utilisateur; un dispositif (5) de reproduction audio configuré pour produire un premier signal sonore (S1) comprenant une bande sonore, un second signal sonore (S2) ayant une première fréquence (f1) et audible d'une oreille de l'utilisateur, un troisième signal sonore (S3) ayant une seconde fréquence (f2) et audible de l'autre oreille de l'utilisateur, et un quatrième signal sonore (S4) comprenant un exposé oratoire; et
une unité de contrôle (7) configurée pour contrôler le module de réalité virtuelle (4) **caractérisé en ce que**:
le dispositif de visualisation (2) projette la séquence d'images (I1) et le dispositif (5) de reproduction audio produit le premier signal sonore (S1), le second signal sonore (S2), le troisième signal sonore (S3) et le quatrième signal sonore (S4) pendant une période de stimulation comportant une portion initiale (ti), portion médiane (tm) et une portion finale (tf); et
**en ce que** le dispositif de visualisation (2) produit en outre un signal d'induction (S5) pendant la portion initiale (ti), le signal d'induction (S5) comprenant une projection d'un signal lumineux (L) superposé à la séquence d'images (I1) ou un son bi-canal se déplaçant rythmiquement et selon un mouvement de va-et-vient entre une première et une deuxième zone de l'espace entourant l'utilisateur (10).

2. Le système selon la revendication 1,
dans lequel le module de réalité virtuel est configuré pour projeter un objet virtuel (8a) se déplaçant entre une première et une deuxième zone d'une surface de visualisation dudit dispositif de visualisation.

3. Le système selon la revendication 1 ou 2,
ledit module de réalité virtuel étant configuré pour générer un son bi-canal simulant une source sonore virtuelle se déplaçant rythmiquement et selon un mouvement de-vas-et-viens entre une première et une deuxième zone de l'espace entourant l'utilisateur.

4. Le système selon l'une des revendications 1 à 3,
dans lequel l'unité de contrôle (7) et au moins une partie du module de réalité virtuelle (1) sont compris dans un appareil portable.

5. Le système selon l'une des revendications 1 à 4,
dans lequel, pendant la portion médiane (tm), la production de la séquence d'images (I1) permet une interaction de l'utilisateur avec la séquence d'images (I1).

6. Le système selon la revendication 5,
dans lequel des caractéristiques de la séquence d'images (I1), notamment l'intensité lumineuse, sont modifiées par l'interaction de l'utilisateur.

7. Le système selon l'une des revendications 1 à 6,
dans lequel la séquence d'images (I1) comprend un ou une pluralité d'objets qui apparaissent et/ou disparaissent suite à un input de l'utilisateur (10).

8. Le système selon l'une des revendications 1 à 7,
dans lequel le signal lumineux (L) se déplace selon un mouvement aléatoire, prédéterminé ou rythmiquement entre une première et une deuxième zone de la séquence d'images (I1).

9. Le système selon l'une des revendications 1 à 8,
dans lequel le cinquième signal sonore (S5) se déplace selon un mouvement aléatoire, prédéterminé ou selon un mouvement de va-et-vient entre une première et une deuxième zone dans l'espace entourant l'utilisateur.

10. Le système selon l'une des revendications 1 à 9,
dans lequel le dispositif (1) de réalité virtuelle comprend au moins un capteur configuré pour mesurer un paramètre vital (HR) de l'utilisateur; et comprenant la détermination et le suivi du paramètre vital (HR) pendant au moins une portion de la période prédéterminée.

11. Support informatique comprenant des portions de code d'un programme d'application destinées à être exécutées par l'unité de contrôle (7) du système selon l'une des revendications 1 à 10, de manière à projeter la séquence d'images (I1) et de produire le premier signal sonore (S1), le second signal sonore (S2), le troisième signal sonore (S3) et le quatrième signal sonore (S4) pendant une période de stimulation comportant une portion initiale (ti), portion médiane (tm) et une portion finale (tf); et
de produire un signal d'induction (S5) pendant la portion initiale (ti), le signal d'induction (S5) comprenant une projection d'un signal lumineux (L) superposé à la séquence d'images (I1) ou un son bi-canal se déplaçant rythmiquement et selon un mouvement de va-et-vient entre une première et une deuxième zone de l'espace entourant l'utilisateur (10).

## Patentansprüche

1. Virtual-Reality-System, umfassend:
eine Virtual-Reality-Vorrichtung (1) mit einem Virtual-Reality-Modul (4) mit einer Anzeigevorrichtung (2), welche konfiguriert ist, um eine Bildsequenz (I1) in das Sichtfeld eines Benutzers zu projizieren; eine Audiowiedergabevorrichtung (5), welche konfiguriert ist, um eine erstes Tonsignal (S1) mit einer Tonspur zu erzeugen, ein zweites Tonsignal (S2) mit einer ersten Frequenz (f1) und für das Ohr eines Benutzers hörbar, ein drittes Tonsignal (S3) mit einer zweiten Frequenz (f2) und für das andere Ohr des Benutzers hörbar, und ein viertes Tonsignal (S4), welches eine oratorische Präsentation umfasst; und
eine Steuereinheit (7), welche zum Steuern des Virtual-Reality-Moduls (4) konfiguriert ist;
**dadurch gekennzeichnet, dass**
die Anzeigevorrichtung (2) die Bildsequenz (11) projiziert und die Audiowiedergabevorrichtung (5) das erste Tonsignal (S1), das zweite Tonsignal (S2), das dritte Tonsignal (S3) und das vierte Tonsignal (S4) während einer Stimulationsperiode mit einem Anfangsabschnitt (ti), einem mittleren Abschnitt (tm) und einem Schlussabschnitt (tf) erzeugt; und
dass die Anzeigevorrichtung (2) zudem ein Induktionssignal (S5) während dem Anfangsabschnitt (ti) erzeugt, wobei das Induktionssignal (S5) umfasst: eine Projektion eines der Bildsequenz (I1) überlagerten Lichtsignals (L) oder einen Zweikanal-Sound, der sich rhythmisch und in einer Hin-und-her-Bewegung zwischen einem ersten und einem zweiten Bereich des den Benutzer (10) umgebenden Raums bewegt.

2. System gemäss Anspruch 1, worin das Virtual-Reality-Modul konfiguriert ist, um ein virtuelles Objekt (8a) zu projizieren, welches sich zwischen einem ersten und einem zweiten Bereich einer Sichtfläche der besagten Anzeigevorrichtung bewegt.

3. System gemäss Anspruch 1 oder 2, wobei das besagte Virtual-Reality-Modul konfiguriert ist, um einen Zweikanal-Sound zu erzeugen, der eine virtuelle Soundquelle simuliert, welche sich rhythmisch und in einer Hin-und-her-Bewegung zwischen einem ersten und einem zweiten Bereich des den Benutzer umgebenden Raums bewegt.

4. System gemäss einem der Ansprüche 1 bis 3, worin die Steuereinheit (7) und mindestens ein Teil des Virtual-Reality-Moduls (1) in einem tragbaren Gerät enthalten sind.

5. System gemäss einem der Ansprüche 1 bis 4, worin, während dem mittleren Abschnitt (tm), die Erzeugung der Bildsequenz (I1) eine Interaktion des Benutzers mit der Bildsequenz (I1) ermöglicht.

6. System gemäss Anspruch 5, worin die Eigenschaften der Bildsequenz (I1), insbesondere die Lichtintensität, durch die Interaktion des Benutzers geändert werden.

7. System gemäss einem der Ansprüche 1 bis 6, worin die Bildsequenz (I1) ein oder mehrere Objekte umfasst, welche infolge einer Eingabe vom Benutzer (10) erscheinen und/oder verschwinden.

8. System gemäss einem der Ansprüche 1 bis 7, worin sich das Lichtsignal (L) in einer zufälligen, vorbestimmten oder rhythmischen Bewegung zwischen einem ersten und einem zweiten Bereich der Bildsequenz (I1) bewegt.

9. System gemäss einem der Ansprüche 1 bis 8, worin das fünfte Tonsignal (S5) in einer zufälligen, vorbestimmten oder Hin-und-her-Bewegung zwischen einem ersten und einem zweiten Bereich im den Benutzer umgebenden Raum bewegt.

10. System gemäss einem der Ansprüche 1 bis 9, worin die Virtual-Reality-Vorrichtung (1) mindestens einen Sensor umfasst, der zum Messen eines Vitalparameters (HR) des Benutzers konfiguriert ist; und umfassend das Bestimmen und Überwachen des Vitalparameters (HR) für mindestens einen Teil der vorbestimmten Periode.

11. Computerträger umfassend Codeabschnitte eines Anwendungsprogramms, welche von der Steuereinheit (7) des Systems gemäss einem der Ansprüche 1 bis 10 ausgeführt werden sollen, um die Bildsequenz (I1) zu projizieren und das erste Tonsignal (S1), das zweite Tonsignal (S2), das dritte Tonsignal (S3) und das vierte Tonsignal (S4) während einer Stimulationsperiode mit einem Anfangsabschnitt (ti), einem mittleren Abschnitt (tm) und einem Schlussabschnitt (tf) zu erzeugen; und
ein Induktionssignal (S5) während dem Anfangsabschnitt (ti) zu erzeugen, wobei das Induktionssignal (S5) umfasst: eine Projektion eines der Bildsequenz (I1) überlagerten Lichtsignals (L) oder einen Zweikanal-Sound, der sich rhythmisch und in einer Hin-und-her-Bewegung zwischen einem ersten und einem zweiten Bereich des den Benutzer (10) umgebenden Raums bewegt.

## Claims

1. Virtual reality system, comprising:
A virtual reality device (1) including a virtual reality module (4) including a display device (2) configured to project an image sequence (11) in the visual field of a user; an audio playback device (5) configured to produce a first sound signal (S1) comprising a soundtrack, a second sound signal (S2) having a first frequency (f1) and being audible by one ear of the user, a third soundtrack (S3) having a second frequency (f2) and being audible by the other ear of the user, and a fourth soundtrack (S4) comprising a spoken presentation; and
A control unit (7) configured to control the virtual reality module (4) **characterized in that**:
the display device (2) projects the image sequence (11) and the audio playback device (5) produces the first sound signal (S1), the second sound signal (S2), the third sound signal (S3) and the fourth sound signal (S4) during a period of stimulation comprising an initial portion (ti), a median portion (tm) and a final portion (tf); and
in which the display device (2) further produces an induction signal (S5) during the initial portion (ti), the induction signal (S5) comprises a projection of a light signal (L) superimposed on the image sequence (11) or a dual-channel sound displacing rythmically and in a to-and-fro movement between the first and a second area in the space surrounding the user (10).

2. The system according to claim 1,
wherein the virtual reality module is configured to project a virtual object (8a) being displaced between a first and a second area of a display surface of said display device.

3. The system according to claim 1 or 2,
said virtual reality module is configured to generate a dual-channel sound simulating a virtual sound source being displaced rhythmically in a to-and-fro movement between a first and a second area of space surrounding the user.

4. The system according to one of the claims 1 to 3,
wherein the control unit (7) and at least part of the virtual reality module (1) are included in a portable device.

5. The system according to one of the claims 1 to 4, wherein, during the median portion (tm), the production of the image sequence (11) allowing an interaction of the user with the image sequence (11).

6. The system according to claim 5,
wherein characteristics of the image sequence (I1), notably the light intensity, are changed by the interaction of the user.

7. The system according to one of the claims 1 to 6,
wherein the image sequence (I1) includes one or a plurality of objects that appear and/or disappear as a result of an input of the user (10).

8. The system according to one of the claims 1 to 7,
wherein the light signal (L) is displaced in a random or predetermined movement or rhythmically between a first and a second area of the image sequence (I1).

9. The system according to one of the claims 1 to 8,
wherein the fifth sound signal (S5) is displaced in a random or predetermined movement or in a to-and-fro movement between a first and a second area in the space surrounding the user.

10. The system according to one of the claims 1 to 9,
wherein the virtual reality device (1) comprises at least one sensor configured to measure a vital parameter (HR) of the user; and comprising the determination and the follow-up of the vital parameter (HR) during at least one portion of the predetermined period.

11. Computer medium including portions of code of an application program intended to be executed by the control unit (7) of the system according to one of the claims 1 to 10, so as to project the image sequence (11) and to produce the first sound signal (S1), the second sound signal (S2), the third sound signal (S3) and the fourth sound signal (S4) during a period of stimulation including an initial portion (ti), a median portion (tm) and a final portion (tf); and
to produce an induction signal (S5) during the initial portion (ti), the induction signal (S5) including a projection of a light signal (L) superimposed on the image sequence (I1) or a dual-channel sound moving rythmically and according to a to-and-fro movement between a first and a second area of the space surrounding the user (10).
